# EUROPÄISCHE PATENTSCHRIFT

(11) **EP 4 394 423 B1**
(45) Veröffentlichungstag und Bekanntmachung des Hinweises auf die Patenterteilung: **12.02.2025**
(21) Anmeldenummer: 22216784.3
(22) Anmeldetag: 27.12.2022
(51) Int. Cl.: G01R 33/48, G01R 33/565, G01R 33/56, G01R 33/483

(54) **VORRICHTUNG UND VERFAHREN ZUR ECHTZEIT-3D-VERZERRUNGSKORREKTUR VON MAGNETRESONANZABBILDUNGEN**
APPARATUS AND METHOD FOR REAL-TIME 3D DISTORTION CORRECTION OF MAGNETIC RESONANCE IMAGING
APPAREIL ET PROCÉDÉ DE CORRECTION DE DISTORSION 3D EN TEMPS RÉEL D'IMAGERIE PAR RÉSONANCE MAGNÉTIQUE

(43) Veröffentlichungstag der Anmeldung: 03.07.2024
(73) Patentinhaber: Siemens Healthineers AG, 91301 Forchheim (DE)
(72) Erfinder: Maier, Florian, 91054 Buckenhof (DE); Requardt, Martin, 90425 Nürnberg (DE)
(74) Vertreter: Siemens Healthineers Patent Attorneys

(56) Entgegenhaltungen:
- US-B2- 9 848 799
- ELGORT D R ET AL: "A Review of Technical Advances in Interventional Magnetic Resonance Imaging", ACADEMIC RADIOLOGY, ELSEVIER, AMSTERDAM, NL, vol. 12, no. 9, September 2005 (2005-09-01), pages 1089 - 1099, XP025311575, ISSN: 1076-6332, [retrieved on 20050901], DOI: 10.1016/J.ACRA.2005.06.003
- HEIDT TIMO ET AL: "Real-time magnetic resonance imaging - guided coronary intervention in a porcine model", SCIENTIFIC REPORTS, vol. 9, no. 1, 17 June 2019 (2019-06-17), XP093046357, Retrieved from the Internet <URL:https://www.nature.com/articles/s41598-019-45154-7> [retrieved on 20230512], DOI: 10.1038/s41598-019-45154-7

## Beschreibung

*Unabhängig vom grammatikalischen Geschlecht eines bestimmten Begriffes sind Personen mit männlicher, weiblicher oder anderer Geschlechteridentität mit umfasst.*

Die Erfindung betrifft einen Magnetresonanztomographen und ein Verfahren für eine Echtzeitverfolgung eines medizinischen Instruments im Rahmen einer interventionellen MR-Bildgebung.

Bei minimalinvasiven medizinischen Prozeduren werden medizinische Instrumente, beispielsweise Katheter und/oder Eingriffsnadeln, in einen Patienten eingeführt, wobei üblicherweise eine Bildüberwachung der Prozedur erfolgt. Die Bildüberwachung ermöglicht die Aufnahme von Bildern, in denen das medizinische Instrument in Bezug zu seiner anatomischen Umgebung sichtbar gemacht wird. Während klassischerweise Röntgenbildgebung zur Bildüberwachung minimalinvasiver medizinischer Eingriffe verwendet wurde, insbesondere Fluoroskopie, wurde inzwischen auch vorgeschlagen, Magnetresonanzgeräte, mithin die Magnetresonanzbildgebung (MR-Bildgebung), zur Bildüberwachung einzusetzen. Dies wird typischerweise als interventionelle MR-Bildgebung bezeichnet.

Dabei sind die meisten medizinischen Instrumente metallisch oder aus anderen nicht mit der Magnetresonanzaufnahme der Protonen im Wasser oder Fett des Körpers unmittelbar abzubildenden Materialien. Stattdessen finden indirekte Verfahren zur Abbildung Anwendung, bei denen indirekte Auswirkungen bzw. Artefakte zur Abbildung genutzt werden. Für eine Echtzeitverfolgung sind aber kurze Wiederholungszeiträume nötig, die nur schlechte Bildqualität liefert. Darüber hinaus sind bei der Magnetresonanztomographie geometrische Verzerrungen üblich, die im Gegensatz zu der geometrietreuen Abbildung durch Röntgenstrahlen das Verfolgen der dünnen Instrumente erschwert.

Aus dem Artikel Elgort D. R. et al., "A Review of Technical Advances in Interventional Magnetic Resonance Imaging", Academic Radiology, 2005, Bd. 12, Nr. 9, Seiten 1089-1099, ist ein Verfahren zum Abbilden eines medizinischen Instruments in Echtzeit bekannt, wobei erste und zweite zueinander orthogonale Schichten aufgenommen werden.

Es ist daher eine Aufgabe der vorliegenden Erfindung, das Verfolgen eines medizinischen Instruments bei einer Intervention zu verbessern.

Die Aufgabe wird durch einen Magnetresonanztomographen nach Anspruch 1 und ein Verfahren zum Betrieb des Magnetresonanztomographen nach Anspruch 8 gelöst.

Der erfindungsgemäße Magnetresonanztomograph ist zum Abbilden eines medizinischen Instruments auf einer Trajektorie in Echtzeit ausgebildet. Der Magnetresonanztomograph weist eine Steuerung auf, die die für eine Bildaufnahme mit dem Magnetresonanztomographen erforderlichen Aktivitäten koordiniert. Die Steuerung kann dabei auch in Untereinheiten gegliedert sein, beispielsweise in eine Gradienten- und Hochfrequenzsteuerung zur Steuerung von Vorgängen in Realzeit, eine Bedienschnittstelle zur Interaktion mit einem Bediener und einen Bildrekonstruktionsrechner mit hoher Rechenkapazität.

Die Steuerung ist ausgelegt, den Magnetresonanztomographen so anzusteuern, dass eine erste Schicht mittels einer 3D-Encodierung erfasst wird. Als Schicht, auf Englisch auch als "Slab" bezeichnet, wird dabei ein 3-dimensionales Volumen angesehen, dass auch eine Dicke im Sinne der Magnetresonanzabbildung aufweist, also nicht nur aus einer einzelnen zweidimensionalen Anordnung aus Voxeln der Magnetresonanzabbildung in einer Fläche besteht, sondern zumindest zwei Lagen von Voxeln übereinander, vorzugsweise mehreren Lagen. So ist es möglich, dass das medizinische Instrument trotz Verzerrungen der Abbildung vollständig erfasst werden kann.

Die Steuerung ist dabei ausgelegt, eine Position der ersten Schicht in Abhängigkeit von der Trajektorie und die Schichtdicke in Abhängigkeit von einer vorbestimmten Verzerrung des Magnetresonanztomographen bei einer Abbildung so auszuwählen bzw. für die Erfassung der ersten Schicht Parameter derart einzustellen, dass die Trajektorie bzw. das medizinische Instrument durch die erste Schicht vollständig erfasst wird. Die Position umfasst in diesem Zusammenhang sowohl den Ort als auch Ausrichtung der Schicht. Die Trajektorie kann beispielsweise durch die Intervention vorgegeben sein, durch Koordinaten, die bei der Planung bestimmt wurden. Es ist aber auch denkbar, dass die Trajektorie vorher ermittelt wurde, beispielsweise indem die Position und/oder Ausrichtung des medizinischen Instruments durch eine Abbildung erfasst wird. Unter Kenntnis der Position der Trajektorie und der Verzerrungen der Abbildung durch den Magnetresonanztomographen kann die Steuerung ein Volumen, beispielsweise einen Quader aus Voxeln bestimmen, in dem die Trajektorie unter Berücksichtigung der Verzerrung liegt. Vorzugsweise ist die Schichtdicke dabei so ausgelegt, dass die Schicht nur die Trajektorie umfasst, also keine oder nur wenige Lagen an Voxeln aufweist, die nicht von der Trajektorie durchstoßen werden bzw. keine zu der Trajektorie zugehörige Voxel aufweisen, um die 3D-Encodierung zu beschleunigen.

Weiterhin ist die Steuerung ausgelegt, eine zweite Schicht in der beschriebenen Weise mittels einer 3D-Encodierung zu erfassen, wobei die Steuerung eine Position der zweiten Schicht in Abhängigkeit von der Trajektorie und die Schichtdicke in Abhängigkeit von einer vorbestimmten Verzerrung des Magnetresonanztomographen bei einer Abbildung so auswählt, dass die Trajektorie durch die zweite Schicht vollständig erfasst wird.

Die Steuerung ist darüber hinaus ausgelegt, die Positionen der ersten Schicht und der zweiten Schicht derart auszuwählen, dass die erste Schicht und die zweite Schicht einen Winkel größer 20 Grad, 40 Grad oder 60 Grad einschließen, kleiner oder gleich 90 Grad, und die Trajektorie des medizinischen Instruments und/oder das medizinische Instrument in einer Schnittmenge bzw. einem Schnittvolumen der ersten Schicht und der zweiten Schicht liegt. Denkbar ist beispielsweise, dass die Steuerung die Koordinaten der ersten Schicht um den Winkel um die Trajektorie rotiert, um die Position der zweiten Schicht zu erhalten.

Die jeweilige Schicht liefert in Richtung der lateralen bzw. flächigen Erstreckung auch eine Abbildung der weiteren Umgebung der Trajektorie. Vorzugsweise entspricht dabei die Erstreckung der Schicht jeweils der Ausrichtung der zweidimensionalen Abbildungen, die nachfolgend dem Nutzer zur Navigation dargestellt werden, sodass auch die weitere Umgebung zur Orientierung dargestellt werden kann. Durch Darstellung von Abbildungen aus beiden Schichten, vorzugsweise unter Kombination von Bilddaten beider Schichten, kann auf vorteilhafte Weise eine hohe Wiederholrate für die Trajektorie erzielt werden und gleichzeitig eine weitere Umgebung dargestellt werden.

Schließlich ist die Steuerung ausgelegt, eine Folge zweidimensionaler Abbildungen des medizinischen Instruments in einer vorbestimmten Ebene zu erzeugen. Die Trajektorie und damit das medizinische Gerät liegen in dieser Ebene. Die Abbildungen werden aus der ersten Schicht und der zweiten Schicht erzeugt, bzw. bei einer Wiederholung aus einer Mehrzahl an ersten Schichten und zweiten Schichten. Vorzugsweise ist die vorbestimmte Ebene parallel zu der ersten Schicht oder der zweiten Schicht, kann aber auch einen kleinen Winkel mit der ersten Schicht bzw. der zweiten Schicht einschließen, um möglichst viel der Umgebung der Trajektorie wiedergeben zu können. Vorzugsweise wird auch eine weitere Abbildung von der Steuerung erzeugt und dargestellt, deren Darstellungsebene parallel oder unter einem kleinen Winkel zu der anderen Schicht liegt und somit Darstellung der Trajektorie bzw. Verfolgung des medizinischen Instruments unter unterschiedlichen Perspektiven ermöglicht.

Die Steuerung ist darüber hinaus ausgelegt ist, die Folge zweidimensionaler Abbildungen schließlich an einen Nutzer auszugeben, beispielsweise auf einem Bildschirm. Die Ausgabe erfolgt dabei möglichst unmittelbar nach dem Erzeugen der Abbildung, um eine Darstellung des medizinischen Instruments bzw. der Trajektorie in Echtzeit bzw. nahezu Echtzeit zu gewährleisten. Insbesondere erfolgt das Aufnehmen einer ersten Schicht, Erzeugen der zweidimensionalen Abbildung in der Ebene und deren Ausgabe vorzugsweise bevor oder während die zweite Schicht aufgenommen und die zweidimensionale Abbildung erzeugt wird.

In einer bevorzugten Ausführungsform werden jeweils zweidimensionale Abbildungen abwechselnd aus Aufnahmen der ersten Schicht und der zweiten Schicht erzeugt und dargestellt, um die Trajektorie aus verschiedenen Perspektiven bzw. Blickrichtungen im Wesentlichen senkrecht zu der jeweiligen Schicht zu erzeugen. Vorzugsweise wird dabei der Bereich der Abbildungen, der der Schnittmenge bzw. dem Schnittvolumen der beiden Schichten entspricht, jeweils mit dem Erfassen der ersten Schicht und der zweiten Schicht aktualisiert bzw. dargestellt.

Auf vorteilhafte Weise ermöglicht es die Aufnahme der beiden Schichten, unter einem Winkel zueinander ausgerichtet, mit der Trajektorie innerhalb der Schichten, Abweichungen der Bewegung des medizinischen Instruments von der Trajektorie im Raum schnell zu erfassen und dem Benutzer einschließlich der weiteren Umgebung darzustellen. Insbesondere ist die Bildwiderholungsrate für die Trajektorie in der Schnittmenge der Schichten verdoppelt im Vergleich zu einem Magnetresonanztomographen, der abwechselnd Schichten unter einem Winkel zueinander angeordnet aufnimmt, um eine Bewegung dreidimensional zu verfolgen, aber dann nur jeweils die zweidimensionale Abbildung aus der Schicht erzeugt, die im Wesentlichen parallel zur Darstellungsebene ausgerichtet ist. Der Bereich der Schnittmenge beider Schichten mit der Trajektorie wird also vorzugsweise mit jeder Erfassung einer der beiden Schichten auch in der Abbildung, die parallel zur anderen Schicht ausgerichtet ist, aktualisiert und so für diesen Bereich die Bildwiederholrate verdoppelt.

Das erfindungsgemäße Verfahren teilt die Vorteile der erfindungsgemäßen Vorrichtung.

Weitere vorteilhafte Ausführungsformen sind zu den Unteransprüchen angegeben.

In einer möglichen Ausführungsform des erfindungsgemäßen Magnetresonanztomographen ist die Steuerung des Magnetresonanztomographen ausgelegt, ein Entzerren der vorbestimmten Verzerrung der ersten Schicht und/oder der zweiten Schicht vorzunehmen. Die Verzerrung einer Bilderfassung des Magnetresonanztomographen kann durch Inhomogenitäten des statischen Magnetfeldes, der Gradientenfelder und dynamisch durch Wirbelströme verursacht werden und ist insofern durch den Magnetresonanztomographen vorbestimmt. Die vorbestimmte Verzerrung kann dabei typspezifisch für den Magnetresonanztomographen durch Berechnung bei der Entwicklung oder Testmessungen bestimmt werden. Auch ist es denkbar, dass die vorbestimmte Verzerrung individuell für einzelne Geräte bei der Installation oder vor einer Bilderfassung durch Kalibriermessungen ermittelt wird. Für die ermittelte Verzerrung als lineare Abbildung, beispielsweise definiert durch eine Matrix, gibt es eine entsprechende Umkehrfunktion, die sich aus der vorbestimmten Verzerrung mit den Mitteln der lineare Algebra bestimmen lässt, um die erfasste erste und/oder zweite Schicht derart zu entzerren, dass die Trajektorie bzw. das medizinische Gerät geometrisch korrekt dargestellt wird, insbesondere bei einer geraden bzw. linearen Trajektorie diese als Teilstück einer Gerade widergibt.

Auf vorteilhafte Weise wird durch das Entzerren die Geometrie der erfassten 3D-Daten und damit auch der daraus erzeugten 2-dimensionalen Abbildung so korrigiert, dass dem Benutzer eine unverzerrte Darstellung des medizinischen Instruments und der Trajektorie geboten wird und so die intuitive Navigation des Instruments erleichtert wird.

In einer denkbaren Ausführungsform des erfindungsgemäßen Magnetresonanztomographen ist der Magnetresonanztomograph weiterhin ausgelegt, eine Mittelung der ersten Schicht und/oder zweiten Schicht nach dem Entzerren vorzunehmen. Unter Mittelung im Sinne der Erfindung zu verstehen, dass durch die Mittelung über mehrere benachbarte Voxel der 3D-Magnetresonanzaufnahme ein Signal-zu-Rausch-Verhältnis (SNR) der Bilderfassung verbessert wird. Im einfachsten Fall und ursprünglicher Wortbedeutung ist die Mittelung ein Binning, ein Zusammenfassen benachbarter Voxel zu virtuellen, größeren Voxeln durch Summieren der Werte. Gleichzeitig nimmt aber die räumliche Auflösung ab. Mit der reduzierten Auflösung ist aber insbesondere ein Ergebnis des Entzerrens deutlich schlechter. Es sind aber auch andere Formen der Mittelung, beispielsweise mit gewichtetem Summieren denkbar.

In einer möglichen Ausführungsform des erfindungsgemäßen Magnetresonanztomographen ist die Steuerung deshalb weiterhin ausgelegt, das Entzerren mit einer Auflösung größer als eine Auflösung der späteren zweidimensionalen Abbildung vorzunehmen.

Auf vorteilhafte Weise erfolgt bei dem erfindungsgemäßen Magnetresonanztomographen deshalb das Entzerren, bevor durch eine Mittelung bzw. ein Binning die Auflösung reduziert wird, sodass eine Lage des medizinischen Instruments genauer dargestellt werden kann. Die Mittelung erfolgt erst anschließend, um das SNR der korrigierten Aufnahme zu verbessern. Da die Mittelung selbst keine umkehrbare Abbildung bzw. Funktion ist, ist die Reihenfolge entscheidend und verändert das Ergebnis. Mit einer größeren Auflösung der Magnetresonanzdaten können diese mit besserem Ergebnis entzerrt werden. Das verschlechterte SNR, das mit der größeren Auflösung bei identischer Messzeit erreicht wird, kann dann durch die nachgeschaltete Mittelung wieder verbessert werden.

In einer denkbaren Ausführungsform des erfindungsgemäßen Magnetresonanztomographen ist die Steuerung weiterhin ausgelegt, eine dritte Schicht enthaltend das medizinische Instrument mit einer 3D-Encodierung zu erfassen. Vorzugsweise wird die dritte Schicht mit einer höheren Auflösung und/oder längeren Integrationszeit als die erste Schicht und/oder zweite Schicht erfasst, sodass insbesondere die Umgebung des medizinischen Instruments genauer erfasst und dargestellt werden kann. Die Steuerung ist dabei ausgelegt, beim Erzeugen der Folge von zweidimensionalen Abbildungen die Abbildungen in Abhängigkeit von der dritten Schicht zu erzeugen. Vorzugsweise wird dabei die Aufnahme der dritten Schicht dazu genutzt, eine Umgebung der Trajektorie mit höherer Auflösung und/ oder besserem SNR in der 2-dimensionalen Abbildung widerzugeben. Die Umgebung der Trajektorie verändert sich während der Handhabung des medizinischen Instruments kaum, sodass es denkbar ist, für die Widergabe der Umgebung in der 2-dimensionalen Abbildung Daten der Aufnahme der dritten Schicht zu verwenden, insbesondere auch in mehreren aufeinanderfolgenden Abbildungen, ohne dazwischen die Aufnahme der dritten Schicht zu wiederholen bzw. zu aktualisieren. Es ist auch denkbar, durch Segmentierung hier die Lage einzelner Organe oder Gefäße in der ersten und/oder zweiten Schicht zu erfassen und entsprechend die Lage in der Aufnahme in der dritten Schicht für die 2-dimensionale Abbildung zu korrigieren.

Auf vorteilhafte Weise erlaubt die Kombination einer Bilderfassung der Umgebung mit hoher Bildqualität, aber niedriger Wiederholrate, mit schnellen Aufnahmen der ersten und zweiten Schicht mit dem medizinischen Instrument eine Verfolgung des Instruments in Echtzeit bei hoher Auflösung umgebender Organe.

In einer möglichen Ausführungsform des erfindungsgemäßen Magnetresonanztomograph ist die Trajektorie, die mit der ersten Schicht und der zweiten Schicht erfasst wird, eine gekrümmte Trajektorie bzw. das medizinische Instrument nicht im Wesentlichen linear ausgestaltet, sondern gekrümmt. Wie bereits zu einer Verzerrung durch die Bilderfassung des Magnetresonanztomographen dargelegt, ist es auch denkbar, dass durch eine geeignete Wahl der ersten Schicht und der zweiten Schicht nicht nur ein durch die Magnetresonanzabbildung verzerrtes lineares Instrument vollständig erfasst wird, sondern bei Kenntnis der Geometrie und Lage der Trajektorie bzw. des medizinischen Instruments auch ein gekrümmtes Instrument bzw. eine gekrümmte Trajektorie vollständig erfasst wird. Dazu wird der Quader, der die erste Schicht und oder zweite Schicht begrenzt, so gewählt, dass er das gekrümmte Instrument bzw. die gekrümmte Trajektorie unter Berücksichtigung der Verzerrungen des Magnetresonanztomographen vollständig einschließt.

Auf vorteilhafte Weise sind der erfindungsgemäße Magnetresonanztomograph und das erfindungsgemäße Verfahren so in der Lage, auch ein gekrümmtes medizinisches Instrument bzw. ein entsprechendes Instrument auf einer gekrümmten Trajektorie in Echtzeit zu verfolgen.

In einer denkbaren Ausführungsform des erfindungsgemäßen Magnetresonanztomographen ist die Steuerung ausgelegt, beim Erzeugen der zweidimensionalen Abbildung eine Linearisierung der gekrümmten Trajektorie vorzunehmen. Denkbar ist es dabei, dass die Linearisierung die Verzerrung durch den Magnetresonanztomographen und/oder die geometrische Krümmung der Trajektorie bzw. des medizinischen Instruments korrigiert bzw. linearisiert. Die Korrektur kann dabei in einer Ebene oder auch im Raum erfolgen. Denkbar ist es beispielsweise, dass eine Linearisierung in einer Ebene senkrecht zur Ebene der 2-dimensionalen Abbildung erfolgt.

Auf vorteilhafte Weise ermöglichen der erfindungsgemäße Magnetresonanztomograph und das erfindungsgemäße Verfahren auch gekrümmte medizinische Instrumente bzw. krümmbare medizinische Instrumente auf einer gekrümmten Trajektorie in einer 2-dimensionalen Abbildung schnell und vollständig einem Nutzer darzustellen und eine Navigation mit dem medizinischen Instrument zu verbessern.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanztomographen mit einem erfindungsgemäßen Instrument;
- Fig. 2: eine schematische Darstellung einer von dem erfindungsgemäßen Magnetresonanztomographen in dem erfindungsgemäßen Verfahren erfassten Schicht;
- Fig. 3: eine schematische Darstellung einer von dem erfindungsgemäßen Magnetresonanztomographen in dem erfindungsgemäßen Verfahren erfassten Schicht;
- Fig. 4: eine schematische Darstellung einer relativen Anordnung zweier von dem erfindungsgemäßen Magnetresonanztomographen in dem erfindungsgemäßen Verfahren erfassten Schichten;
- Fig. 5: einen schematischen Ablaufplan eines erfindungsgemäßen Verfahrens.

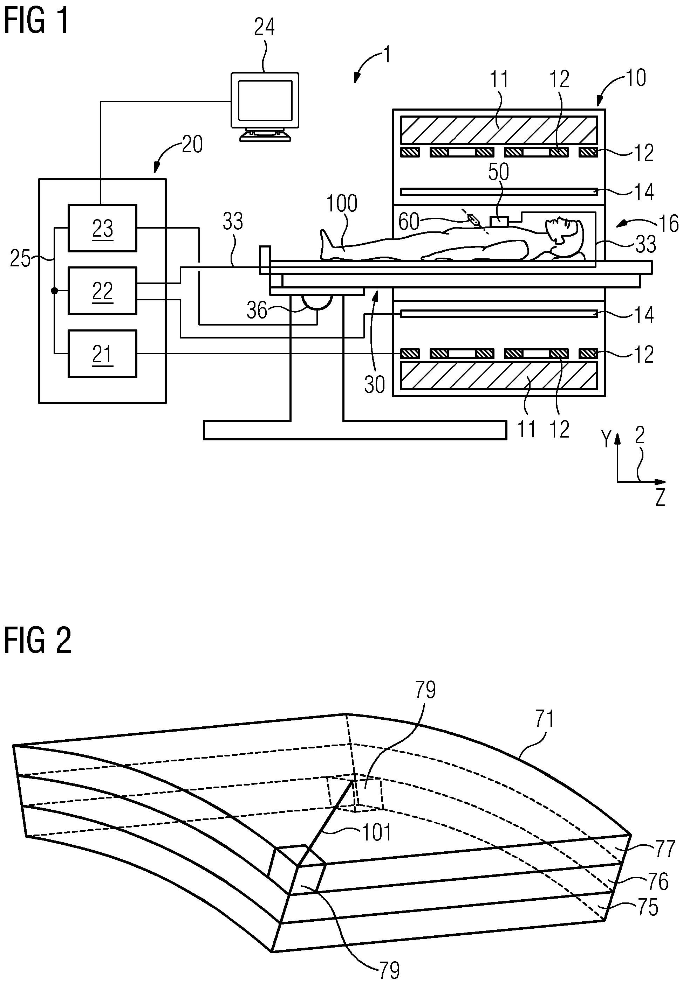

Fig. 1 zeigt eine schematische Darstellung einer Ausführungsform eines erfindungsgemäßen Magnetresonanztomographen 1, wie er auch zum Ausführen des erfindungsgemäßen Verfahrens genutzt wird.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Magnetfeldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 zeitlich und räumlich variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Ein medizinisches Instrument 60, beispielsweise eine Biopsienadel, ist an einem Patienten 100 in einem Patiententunnel 16 angeordnet bzw. wird im Rahmen eines Eingriffs in den Patienten 100 eingeführt und mittels des erfindungsgemäßen Magnetresonanztomographen erfasst und einem Benutzer auf einem Ausgabegerät wie einem Bildschirm 24 dargestellt.

Sowohl das statische B0-Magnetfeld des Feldmagneten 11 als auch die Gradientenfelder der Gradientenspulen 12 weisen Inhomogenitäten bzw. Nichtlinearitäten auf, die zu räumlichen Verzerrungen bei der Abbildung führen.

In Fig. 2 ist schematisch eine erste Schicht 71 einer erfindungsgemäßen Bilderfassung mit einer einbeschriebenen Trajektorie 101 dargestellt. In Fig. 2 ist die reale unverzerrte Geometrie dargestellt, d.h. ein medizinisches Instrument 60 wie eine Biopsienadel, die geometrisch im Wesentlichen eine Teilgerade darstellt, ist hier auch als Teilgerade ohne Krümmung dargestellt. Die erste Schicht 71 einer Bilderfassung durch den Magnetresonanztomographen 1 hingegen ist durch die Verzerrungen der Magnetresonanzabbildung kein Quader mit geraden Kanten, sondern gekrümmt bzw. verbogen.

Die Verzerrungen, die ein Abbildung durch den Magnetresonanztomographen 1 erfährt, sind im Wesentlichen durch den Magnetresonanztomographen 1 und die verwendete Sequenz bestimmt. Sie lassen sich damit für den einzelnen Magnetresonanztomographen 1 für einen typengleiche Geräteserie und mit höherer Genauigkeit für ein individuelles Gerät und eine vorbestimmte Sequenz durch Kalibrierungsmessungen ermitteln. Dies kann beispielsweise durch Abbildung eines Phantoms mit einem Gitter aus magnetresonanzaktiven Proben mit dem Magnetresonanztomographen 1 und der entsprechenden Sequenz geschehen. Denkbar ist auch, eine Verzerrung durch eine Simulation abzuschätzen.

Die ermittelte Verzerrung liefert eine Abbildung einzelner Bildpunkte eines Untersuchungsobjektes in der realen Geometrie auf entsprechende Voxel in einer 3D-Abbildung. Eine entsprechende Umkehrabbildung kann mit den Mitteln der linearen Algebra bestimmt werden.

Für eine vorgegebene Trajektorie 101 lassen sich damit die Orte der einzelnen Punkte der Trajektorie 101 im verzerrten Raum der Voxel ermitteln. So kann auch ein Quader in diesem Voxel-Raum ermittelt werden, der diese verzerrte Trajektorie 101 vollständig enthält bzw. einbeschreibt, gleichzeitig die Anzahl der zu erfassenden Voxel in begrenztem Umfang minimiert. In den meisten Fällen können beispielsweise die Voxel, die dem Anfangs- und Endpunkt der Trajektorie im Voxel-Raum zugeordnet sind, als diagonal gegenüberliegende Eckpunkte des Quaders gewählt werden.

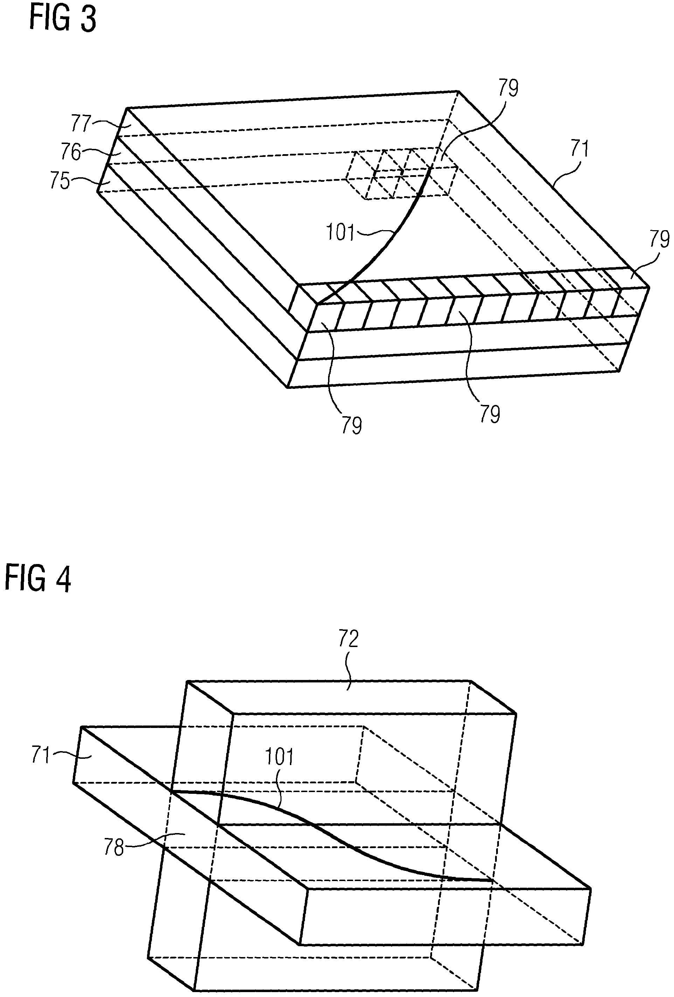

In Fig. 3 ist hingegen die Sicht des Magnetresonanztomographen dargestellt. Die erste Schicht 71 wird durch einen Quader definiert, der mehrere parallele Lagen 75, 76, 77 aus Voxeln in parallelen Ebenen aufweist. Die eigentlich im Beispiel gerade Trajektorie 101 erscheint hier hingegen gekrümmt.

Dabei wird auch ersichtlich, dass es nicht möglich ist, die Trajektorie 101 wegen der Krümmung mit lediglich einer Lage 75, 76, 77 an Voxeln vollständig zu erfassen, wenn die Voxel klein genug sein müssen, um die Trajektorie genau genug für eine Navigation des medizinischen Instruments darzustellen. Die Erfindung schlägt deshalb auch eine 3D-Erfassung der ersten Schicht 71 mit der hier beispielhaften Anzahl von drei Lagen 75, 76, 77 an Voxeln vor. Indem der Quader aber so begrenzt wird, dass er die Trajektorie gerade vollständig einbeschreibt, kann die Anzahl zu erfassender Voxel-Werte verringert und damit die Wiederholungsrate bei gleichem SNR verbessert werden.

Die einzelnen Lagen 75, 76, 77 der Schichten 71, 72 werden vorzugsweise in 3D durch Phasencodierung erfasst. Dazu wird die gesamte Schicht angeregt und vollständig in ihrer Ausdehnung im FoV mit 3D-Phasencodierung erfasst, um Einfaltungen von Voxeln außerhalb des Quaders zu vermeiden. Dies erhöht zwar die Zahl der Voxel und verlängert damit die Erfassungszeit in der Phasencodierung. Dieser Nachteil wird aber durch die wesentlich schnellere Anregung der Schicht 71, 72 gegenüber einer selektiven Anregung des Quaders aufgehoben

Das dargestellte im Wesentlichen kartesische Koordinatensystem, wenn auch im Voxel-Raum verzerrt, ist hier lediglich beispielhaft und wegen der besseren Anschaulichkeit verwendet. Wegen der intuitiven Handhabung für den Nutzer bei der Navigation wird es für das Erfassen der Schichten und damit die daraus erzeugten Abbildungen vorzugsweise genutzt. Die Erfindung lässt sich aber entsprechend auch auf andere Koordinatensysteme anwenden.

In Fig. 4 sind eine erste Schicht 71 und eine zweite Schicht 72 dargestellt. Die einzelnen Lagen 75, 76, 77 der jeweiligen Schicht sind der Übersicht halber nicht mehr einzeln dargestellt. Die erste Schicht 71 und die zweite Schicht 72 weisen ein Schnittvolumen 78 auf, das sich im Inneren von beiden Schichten 71, 72 befindet. Dier erste Schicht 71 und die zweite Schicht 72 sind unter einem Winkel zueinander angeordnet, mit anderen Worten, Normalenvektoren der flächigen Oberflächen, also nicht der Kantenflächen, schließen diesen Winkel ein. Der Winkel kann vorzugsweise 90 Grad betragen, die erste Schicht 71 also senkrecht zu der zweiten Schicht 72 angeordnet sein. Denkbar sind auch andere Winkel größer 30 Grad oder 60 Grad, sodass die Schichten 71, 72 jeweils deutlich unterschiedliche Orientierung in Bezug auf die Trajektorie 101 aufweisen.

Die Trajektorie 101 ist diesem Schnittvolumen 78 einbeschrieben, sodass die Trajektorie 101 sowohl beim Erfassen der ersten Schicht 71 als auch beim Erfassen der zweiten Schicht 72 jeweils miterfasst wird. Auf diese Weise kann die Steuerung 23 eine Position des medizinischen Instruments 60 entlang der Trajektorie 101 in den Abbildungen aus zwei unterschiedlichen Perspektiven parallel zu den Schichten 71, 72 mit doppelter Frequenz aktualisiert werden.

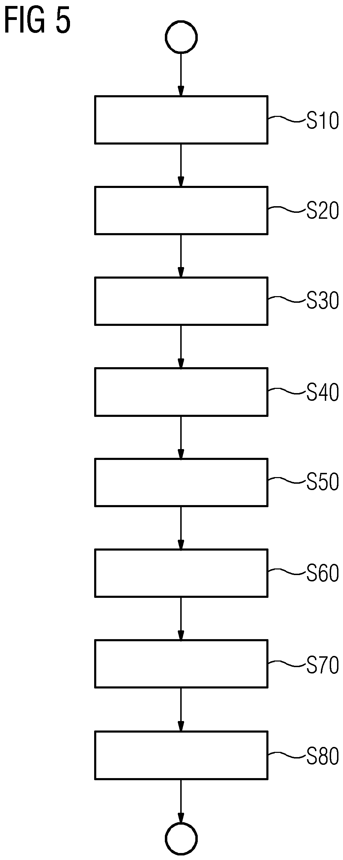

In Fig. 5 ist ein schematischer Ablaufplan einer Ausführungsform des erfindungsgemäßen Verfahrens dargestellt. Das Verfahren wird auf einem erfindungsgemäßen Magnetresonanztomographen 1 ausgeführt.

In einem Schritt S20 wird eine erste Schicht 71 mittels einer 3D-Encodierung von dem Magnetresonanztomographen 1 unter Kontrolle der Steuerung 23 erfasst. Die erste Schicht 71 weist dabei eine Mehrzahl an Lagen 75, 76, 77, in denen Voxel 79 angeordnet sind. Die Position, d.h. Ort und Ausrichtung, und die Schichtdicke der ersten Schicht 71 wird dabei von der Steuerung 23 in Abhängigkeit von der Trajektorie 101 in Abhängigkeit von einer vorbestimmten Verzerrung des Magnetresonanztomographen 1 bei einer Abbildung von der Steuerung 23 gewählt, dass die Trajektorie 101 durch die erste Schicht 71 vollständig erfasst wird. Dazu wird auch auf die Ausführungen zu Fig. 2 und Fig. 3 verwiesen.

Die vorbestimmte Verzerrung kann auch von der Steuerung 23 durch eine Kalibriermessung in einem Schritt S10 vorab bestimmt werden, beispielsweise mit einem Phantom mit einem Gitter aus MR-aktiven Proben an vorbestimmten Positionen.

In einem Schritt S30 wird eine zweite Schicht 72 mittels einer 3D-Encodierung von dem Magnetresonanztomographen 1 unter Kontrolle der Steuerung 23 erfasst. Die Lage der zweiten Schicht 72 und die Schichtdicke wird ebenso in Abhängigkeit von der Trajektorie 101 und in Abhängigkeit von der vorbestimmten Verzerrung des Magnetresonanztomographen 1 so gewählt, dass die Trajektorie 101 durch die zweite Schicht 72 vollständig erfasst wird.

Die Ausrichtung der ersten Schicht 71 und der zweiten Schicht 72 relativ dazu ist, wie in Fig. 4 dargestellt und dazu erläutert, derart von der Steuerung 23 ausgewählt, dass die erste Schicht 71 und die zweite Schicht 72 einen Winkel größer 30 Grad oder 60 Grad einschließen, vorzugsweise gleich 90 Grad, und die Trajektorie 101 des medizinischen Instruments 60 und/oder das medizinische Instrument 60 in einem Schnittvolumen 78 der ersten Schicht 71 und der zweiten Schicht 72 liegt.

In einem Schritt S70 wird von der Steuerung 23 eine Folge zweidimensionaler Abbildung des medizinischen Instruments 60 in einer vorbestimmten Ebene erzeugt. Denkbar ist beispielsweise eine Schnittbild durch die rekonstruierten Voxel 79 des Schnittvolumens 78 entlang dieser Ebene oder eine Projektion auf diese Ebene.

Die Folge von Abbildungen weist dabei Abbildungen auf, die aus der ersten Schicht 71 erzeugt wurden und Abbildungen, die aus der zweiten Schicht 72 erzeugt wurden. Die Darstellungsebene der jeweiligen Abbildung ist dabei im Wesentlichen parallel zu der Schicht, aus der sie erzeugt werden. Vorzugsweise werden dabei die erste Schicht 71 in Schritt S20 alternierend mit der zweiten Schicht S72 in Schritt S30 erfasst. Vorzugsweise wird auch jeweils das Schnittbild gleich aus den erfassten Magnetresonanzdaten der jeweiligen Schicht 71, 72 unmittelbar erzeugt, bevor oder während die jeweils andere Schicht 71, 72 erfasst wird. So können die Abbildungen möglichst zeitnah erzeugt werden. Indem vorzugsweise die Teile der Abbildungen, die das Schnittvolumen 78 darstellen, aus den erfassten Daten bzw. Voxeln der ersten Schicht 71 und der zweiten Schicht 72 aktualisiert werden, können die Darstellungen des medizintechnischen Instruments in den beiden Abbildungen mit doppelter Wiederholrate aktualisiert werden.

In einem Schritt S80 werden dann die zweidimensionalen Abbildungen jeweils von der Steuerung 23 an einen Nutzer ausgegeben, beispielsweise auf einem Bildschirm 24. Das Ausgeben erfolgt dabei möglichst zeitnah an dem Erfassen und Erzeugen der Abbildung aus der jeweiligen Schicht 71, 72. Die Schrittfolge ist vorzugsweise Erfassen S20 einer ersten Schicht 71, Erzeugen S70 der Abbildung der ersten Schicht 71 und unmittelbares Ausgeben S80 der Abbildung der ersten Schicht, bevor oder während in Schritt S30 die zweite Schicht 72 erfasst wird, eine zweidimensionale Abbildung aus der zweiten Schicht 72 in einem Schritt S70 erzeugt und in einem Schritt S980 unmittelbar ausgegeben wird. Vorzugsweise wird diese Abfolge aus Erfassen S20, S30, Erzeugen von zweidimensionalen Abbildungen S70 und Ausgeben S80 für eine vorbestimmte Dauer, für die Dauer eines Steuersignals wie z.B. von einem Fußtaster oder für die Dauer einer Intervention wiederholt.

In einer möglichen Ausführungsform des erfindungsgemäßen Verfahrens werden in einem Schritt S40 die erfassten Magnetresonanzdaten der ersten Schicht 71 oder der zweiten Schicht 72 geometrisch von der Steuerung entzerrt. Der Magnetresonanztomograph 1 weist geometrische Verzerrungen in der Abbildung erfasster Objekte auf. Diese Verzerrungen können durch Inhomogenitäten des statischen B0-Feldes, Nichtlinearitäten der Gradientenfelder oder durch von den Gradienten erzeugten Wirbelströmen verursacht werden.

Die Verzerrungen sind charakteristisch bzw. vorbestimmt für einen jeweiligen Magnetresonanztomographen und die verwendeten Sequenzen. Die Verzerrungen können beispielsweise mit einem Kalibrierscan bestimmt werden, bei dem ein Phantom mit einem Gitter aus MR-aktiven Proben an vorbestimmten Positionen erfasst wird. Die Abweichung der ermittelten Position von der durch das Gitter vorgegebenen Position ergibt die Verzerrung, die in Form einer Matrix angegeben werden kann. Zum Entzerren lässt sich daraus eine Umkehrfunktion in Form einer inversen Matrix bestimmen.

Zum Entzerren in Schritt S40 können beispielsweise aus den erfassten Magnetresonanzdaten der ersten Schicht 71 oder der zweiten Schicht 72 im k-Raum durch Bildrekonstruktion, beispielsweise beruhend auf einer 3D-Fouriertransformation, Bildpunkte bzw. Voxel im Ortsraum ermittelt werden. Deren Koordinaten unterliegen jedoch den Verzerrungen, die von dem Magnetresonanztomographen 1 verursacht werden und können durch ein Anwenden der inversen Matrix auf die Koordinaten korrigiert werden.

Um Echtzeitanforderungen bei einem Verfolgen des medizinischen Instruments 60 besser erfüllen zu können, muss die Bilderfassung mit hoher Rate erfolgen, sodass für eine Signalintegration nur wenig Zeit zur Verfügung steht und damit das SNR gering ist. Dies gilt insbesondere, wenn für eine hohe Ortsauflösung eine große Anzahl an Punkten im k-Raum zu erfassen ist. Das SNR lässt sich jedoch durch eine Mittelung bzw. ein Binning über mehrere Bildpunkte bzw. Voxel verbessern.

Das Entzerren der Geometrie ist aber wiederum mit einem größeren Fehler behaftet, wenn die zugrundeliegenden Voxel im Bildraum größer sind. In einer Ausführungsform des erfindungsgemäßen Verfahrens wird deshalb vorgeschlagen, zunächst das Entzerren auf Daten im Bildraum auszuführen, die eine möglichst hohe Auflösung aufweisen, auch wenn diese verrauscht sind, um eine bessere räumliche Korrektur mit höherer Auflösung zu erzielen, selbst, wenn die Daten für einen Betrachter zunächst keine bessere Darstellung des medizinischen Instruments 60 liefern. Gemäß der Ausführungsform wird anschließend auf die geometrisch entzerrten Voxel bzw. Bildpunkte in einem Schritt S50 eine Mittelung angewendet, um das SNR zu verbessern. Dabei kann die räumliche Dichte der Bildpunkte unverändert bleiben, es ist jedoch auch ein Binning denkbar, bei dem die räumliche Auflösung reduziert wird.

In einer Ausführungsform des erfindungsgemäßen Verfahrens ist es auch denkbar, dass die Trajektorie 101 und/oder das medizinische Instrument 60 selbst geometrisch gekrümmt sind, also nicht Teil einer Geraden sind. Aus Sicht des Magnetresonanztomographen 1 bzw. dessen Steuerung 23 ist der Unterschied zwischen einer Krümmung der Trajektorie durch Verzerrungen der Abbildung und einer geometrischen Krümmung der Trajektorie 101 bzw. des medizinischen Instruments 101 lediglich, dass diese Eigenschaft nicht durch den Magnetresonanztomographen 1 und die Sequenz bestimmt ist. Ist die Krümmung bekannt, kann sie in einem separaten Schritt korrigiert werden, wie es bereits zum Entzerren zu Schritt S40 erläutert ist. Für eine Linearisierung der Krümmung ist beispielsweise eine lineare Abbildung in Form einer Matrix denkbar. Es ist dabei auch denkbar, dass das Entzerren aus Schritt S40 und die Linearisierung gemeinsam in einem Schritt ausgeführt werden, indem beispielsweise die zu S40 beschriebene inverse Matrix mit der Matrix für die Linearisierung multipliziert werden und dann auf die Koordinaten der Bildpunkte bzw. der Voxel aus der MR-Erfassung in Schritt S40 angewendet werden.

Die Krümmung des medizinischen Instruments 60 bzw. der Trajektorie 101 für die Steuerung 23 ist a priori nicht bekannt, insbesondere ist sie keine Konstante für den jeweiligen Magnetresonanztomographen 1 und die Sequenz.

Um in den Schritten S20 und S30 die Dicke und Orientierung der ersten Schicht 71 und der zweiten Schicht 72 zu ermitteln, kann beispielsweise die Trajektorie von extern vorgegeben sein, z.B. durch einen Behandlungsplan, und durch eine Nutzereingabe oder eine elektronische Datenübertragung der Steuerung 23 bekannt sein.

Es ist auch denkbar, dass die Steuerung 23 die Lage des medizinischen Instruments ermittelt, indem sie beispielsweise einen Scan ausführt und durch Segmentierung die Position und Lage des medizinischen Instruments 60 erfasst. Denkbar ist es auch, dazu die Abbildungen der ersten Schicht S20 und/oder der zweiten Schicht in S30 zu nutzen, um die Schichtdicke und/oder Lage für eine darauffolgende Wiederholung der Schritte S20 und/oder S30 anzupassen.

In einer möglichen Ausführungsform wird in einem Schritt S60 eine dritte Schicht in einer 3D-Erfassung von der Steuerung 23 mit dem Magnetresonanztomographen 1 aufgenommen. Die dritte Schicht wird in einer sogenannten through-plane Aufnahme erfasst, mit anderen Worten, die Trajektorie 101 bzw. das medizinische Instrument 60 durchstößt die dritte Schicht in Richtung der Dicke der Schicht, also im Wesentlichen in Richtung der Flächennormale der Schicht. Beispielsweise kann die dritte Schicht orthogonal zur ersten Schicht 71 und zur zweiten Schicht angeordnet sein. Die dritte Schicht kann so auf vorteilhafte Weise eine höher aufgelöste 3D-Abbildung der Umgebung der Trajektorie 101 liefern. Die dritte Schicht kann mit geringer Wiederholrate erfasst werden, da sich eine weitere Umgebung der Trajektorie 101 nur wenig ändert und für eine Verfolgung der medizinischen Instruments 60 in Echtzeit nicht zwingend notwendig ist.

Vorzugsweise wird in Schritt S70 beim Erzeugen der zweidimensionalen Abbildung dies in Abhängigkeit von der dritten Schicht ausgeführt, insbesondere für eine weitere Umgebung der Trajektorie 101. Beispielsweise könnten aus den Bilddaten der ersten Schicht 71 und zweiten Schicht 72 Organe oder Gefäße segmentiert werden und entsprechende Bilddaten aus der Abbildung der dritten Schicht entsprechend eingeblendet werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanztomograph zum Abbilden eines medizinischen Instruments auf einer Trajektorie (101) in Echtzeit, wobei der Magnetresonanztomograph (1) eine Steuerung (23) aufweist, wobei die Steuerung (23) ausgelegt ist, eine erste Schicht (71) mittels einer 3D-Encodierung zu erfassen, wobei die Steuerung die Position der ersten Schicht (71) in Abhängigkeit von der Trajektorie und die Schichtdicke in Abhängigkeit von einer vorbestimmten Verzerrung des Magnetresonanztomographen (1) bei einer Abbildung so wählt, dass die Trajektorie (101) durch die erste Schicht (71) vollständig erfasst wird; die Steuerung (23) ausgelegt ist, eine zweite Schicht (72) mittels einer 3D-Encodierung zu erfassen, wobei Steuerung (23) eine Position der zweiten Schicht (23) in Abhängigkeit von der Trajektorie (101) und die Schichtdicke in Abhängigkeit von einer vorbestimmten Verzerrung des Magnetresonanztomographen (1) bei einer Abbildung so auswählt, dass die Trajektorie (101) durch die zweite Schicht (72) vollständig erfasst wird;
wobei die Steuerung (23) ausgelegt ist, die Position der ersten Schicht (71) und der zweiten Schicht (72) derart auszuwählen, dass die erste Schicht (71) und die zweite Schicht (72) einen Winkel größer 20 Grad einschließen, und die Trajektorie (101) des medizinischen Instruments (60) und/oder das medizinische Instrument (60) in einem Schnittvolumen (78) der ersten Schicht (71) und der zweiten Schicht (72) liegt; die Steuerung (23) ausgelegt ist, eine Folge zweidimensionaler Abbildungen des medizinischen Instruments (60) in einer vorbestimmten Ebene zu erzeugen, wobei die Folge Abbildungen aufweist, die aus der ersten Schicht (71) erzeugt wurden und Abbildungen, die aus der zweiten Schicht (72) erzeugt wurden; und die Steuerung (23) ausgelegt ist, die Folge zweidimensionaler Abbildungen an einen Nutzer auszugeben.

2. Magnetresonanztomograph nach Anspruch 1, wobei die Steuerung (23) des Magnetresonanztomographen (1) ausgelegt ist, ein Entzerren der vorbestimmten Verzerrung der ersten Schicht (71) und/oder der zweiten Schicht (72) vorzunehmen.

3. Magnetresonanztomograph nach Anspruch 2, wobei die Steuerung (23) weiterhin ausgelegt ist, eine Mittelung der ersten Schicht (71) und/oder zweiten Schicht (72) nach dem Entzerren vorzunehmen.

4. Magnetresonanztomograph nach Anspruch 3, wobei die Steuerung (23) weiterhin ausgelegt ist, das Entzerren mit einer Auflösung größer als eine Auflösung der zweidimensionalen Abbildung vorzunehmen.

5. Magnetresonanztomograph nach einem der vorhergehenden Ansprüche, wobei die Steuerung (23) weiterhin ausgelegt ist, eine dritte Schicht enthaltend das medizinische Instrument mit einer 3D-Encodierung zu erfassen, wobei die Steuerung (23) ausgelegt ist, beim Erzeugen der Folge von zweidimensionalen Abbildungen die Abbildungen in Abhängigkeit von der dritten Schicht zu erzeugen.

6. Magnetresonanztomograph nach einem der vorhergehenden Ansprüche, wobei die Trajektorie (101) eine gekrümmte Trajektorie (101) ist.

7. Magnetresonanztomograph nach Anspruch 6, wobei die Steuerung (23) ausgelegt ist beim Erzeugen der zweidimensionalen Abbildung eine Linearisierung der gekrümmten Trajektorie (101) vorzunehmen.

8. Verfahren zum Abbilden eines medizinischen Instruments (60) in Echtzeit mittels eines Magnetresonanztomographen (1) auf einer Trajektorie (101), wobei der Magnetresonanztomograph (1) eine Steuerung (23) aufweist und das Verfahren die Schritte aufweist:
(S20) Erfassen einer ersten Schicht (71) durch den Magnetresonanztomographen (1) mittels einer 3D-Encodierung, wobei eine Position der ersten Schicht (71) in Abhängigkeit von der Trajektorie (101) und die Schichtdicke in Abhängigkeit von einer vorbestimmten Verzerrung des Magnetresonanztomographen (1) bei einer Abbildung so von der Steuerung (23) gewählt wird, dass die Trajektorie (101) durch die erste Schicht (71) vollständig erfasst wird,
(S30) Erfassen einer zweiten Schicht (72) mittels einer 3D-Encodierung durch den Magnetresonanztomographen (1), wobei eine Position der zweiten Schicht (72) in Abhängigkeit von der Trajektorie (101) und die Schichtdicke in Abhängigkeit von einer vorbestimmten Verzerrung des Magnetresonanztomographen (1) bei einer Abbildung so von der Steuerung (23)gewählt wird, dass die Trajektorie (101) durch die zweite Schicht (72) vollständig erfasst wird,
wobei die Position der ersten Schicht (71) und der zweiten Schicht (72) derart ausgewählt sind, dass die erste Schicht (71) und die zweite Schicht (72) einen Winkel größer 20 Grad einschließen, und die Trajektorie (101) des medizinischen Instruments (60) und/oder das medizinische Instrument (60) in einem Schnittvolumen der ersten Schicht (71) und der zweiten Schicht (72) liegt;
(S70) Erzeugen einer Folge zweidimensionaler Abbildungen des medizinischen Instruments (60) in einer vorbestimmten Ebene durch die Steuerung (23), wobei die Folge Abbildungen aufweist, die aus der ersten Schicht (71) erzeugt wurden und Abbildungen, die aus der zweiten Schicht (72) erzeugt wurden;
(S80) Ausgeben der Folge zweidimensionaler Abbildungen an einen Nutzer.

9. Verfahren nach Anspruch 8, wobei das Verfahren weiterhin den Schritt (S40) aufweist, ein Entzerren der vorbestimmten Verzerrung der ersten Schicht (71) und/oder der zweiten Schicht (72) durch die Steuerung (23) vorzunehmen.

10. Verfahren nach Anspruch 9, wobei das Verfahren weiterhin den Schritt (S50) aufweist, eine Mittelung der ersten Schicht (71) und/oder zweiten Schicht (72) nach dem Entzerren durch die Steuerung (23) vorzunehmen.

11. Verfahren nach Anspruch 10, wobei der Schritt (S40) des Entzerrens mit einer Auflösung größer als eine Auflösung der zweidimensionalen Abbildung erfolgt.

12. Verfahren nach einem der Ansprüche 8 bis 11, wobei das Verfahren weiterhin den Schritt (S60) aufweist, eine dritte Schicht enthaltend das medizinische Instrument mit einer 3D-Encodierung durch den Magnetresonanztomographen (1) zu erfassen, wobei in dem Schritt des Erzeugens einer Folge von zweidimensionalen Abbildungen die Abbildungen in Abhängigkeit von der dritten Schicht erzeugt werden.

13. Verfahren nach einem Ansprüche 8 bis 12, wobei die Trajektorie (101) eine gekrümmte Trajektorie (101) ist.

14. Verfahren nach Anspruch 13, wobei in dem Schritt des Erzeugens der zweidimensionalen Abbildung eine Linearisierung der gekrümmten Trajektorie (101) erfolgt.

15. Computerprogrammprodukt mit Befehlen, die bei Ausführung durch ein Magnetresonanztomographen (1) nach einem der Ansprüche 1 bis 7 das Magnetresonanztomographiesystem (1) dazu veranlassen, ein Verfahren nach einem der Ansprüche 8 bis 14 durchzuführen.

## Claims

1. Magnetic resonance tomography device for mapping a medical instrument on a trajectory (101) in real time, wherein the magnetic resonance tomography device (1) has a controller (23),
wherein the controller (23) is designed to capture a first slice (71) by means of 3D encoding, wherein the controller selects the position of the first slice (71) as a function of the trajectory and the slice thickness as a function of a predetermined distortion of the magnetic resonance tomography device (1) during mapping, so that the trajectory (101) is captured in full by the first slice (71);
the controller (23) is designed to capture a second slice (72) by means of 3D encoding, wherein the controller (23) selects a position of the second slice (23) as a function of the trajectory (101) and the slice thickness as a function of a predetermined distortion of the magnetic resonance tomography device (1) during mapping, so that the trajectory (101) is captured in full by the second slice (72);
wherein the controller (23) is designed to select the position of the first slice (71) and the second slice (72) such that the first slice (71) and the second slice (72) confine an angle greater than 20 degrees, and the trajectory (101) of the medical instrument (60) and/or the medical instrument (60) lies in an intersecting volume (78) of the first slice (71) and the second slice (72);
the controller (23) is designed to generate a sequence of two-dimensional images of the medical instrument (60) in a predetermined plane, wherein the sequence contains images that were generated from the first slice (71) and images that were generated from the second slice (72);
and the controller (23) is designed to output the sequence of two-dimensional images to a user.

2. Magnetic resonance tomography device according to claim 1, wherein the controller (23) of the magnetic resonance tomography device (1) is designed to perform an equalisation of the predetermined distortion of the first slice (71) and/or the second slice (72).

3. Magnetic resonance tomography device according to claim 2, wherein the controller (23) is further designed to perform averaging of the first slice (71) and/or second slice (72) after the equalisation.

4. Magnetic resonance tomography device according to claim 3, wherein the controller (23) is further designed to perform the equalisation with a resolution greater than a resolution of the two-dimensional image.

5. Magnetic resonance tomography device according to one of the preceding claims, wherein the controller (23) is further designed to capture a third slice containing the medical instrument with 3D encoding, wherein the controller (23) is designed to generate the images as a function of the third slice during generation of the sequence of two-dimensional images.

6. Magnetic resonance tomography device according to one of the preceding claims, wherein the trajectory (101) is a curved trajectory (101).

7. Magnetic resonance tomography device according to claim 6, wherein the controller (23) is designed to perform a linearisation of the curved trajectory (101) during generation of the two-dimensional image.

8. Method for mapping a medical instrument (60) in real time by means of a magnetic resonance tomography device (1) on a trajectory (101), wherein the magnetic resonance tomography device (1) has a controller (23) and the method contains the steps:
(S20) capture of a first slice (71) by the magnetic resonance tomography device (1) by means of 3D encoding, wherein a position of the first slice (71) is selected by the controller (23) as a function of the trajectory (101) and the slice thickness as a function of a predetermined distortion of the magnetic resonance tomography device (1) during mapping, so that the trajectory (101) is captured in full by the first slice (71),
(S30) capture of a second slice (72) by means of 3D encoding by the magnetic resonance tomography device (1), wherein a position of the second slice (72) is selected by the controller (23) as a function of the trajectory (101) and the slice thickness as a function of a predetermined distortion of the magnetic resonance tomography device (1) during mapping, so that the trajectory (101) is captured in full by the second slice (72),
wherein the position of the first slice (71) and the second slice (72) is selected such that the first slice (71) and the second slice (72) confine an angle greater than 20 degrees, and the trajectory (101) of the medical instrument (60) and/or the medical instrument (60) lies in an intersecting volume of the first slice (71) and the second slice (72);
(S70) generation of a sequence of two-dimensional images of the medical instrument (60) in a predetermined plane by the controller (23), wherein the sequence contains images that were generated from the first slice (71) and images that were generated from the second slice (72);
(S80) output of the sequence of two-dimensional images to a user.

9. Method according to claim 8, wherein the method further contains the step (S40) of the performance by the controller (23) of an equalisation of the predetermined distortion of the first slice (71) and/or the second slice (72).

10. Method according to claim 9, wherein the method further contains the step (S50) of the performance by the controller (23) of averaging of the first slice (71) and/or second slice (72) after the equalisation.

11. Method according to claim 10, wherein the step (S40) of equalisation takes place with a resolution greater than a resolution of the two-dimensional image.

12. Method according to one of claims 8 to 11, wherein the method further contains the step (S60) of capturing a third slice containing the medical instrument with 3D encoding by the magnetic resonance tomography device (1), wherein in the step of the generation of a sequence of two-dimensional images the images are generated as a function of the third slice.

13. Method according to one of claims 8 to 12, wherein the trajectory (101) is a curved trajectory (101).

14. Method according to claim 13, wherein in the step of generation of the two-dimensional image a linearisation of the curved trajectory (101) takes place.

15. Computer program product with commands which during execution by a magnetic resonance tomography device (1) according to one of claims 1 to 7 cause the magnetic resonance tomography system (1) to perform a method according to one of claims 8 to 14.

## Revendications

1. Tomodensitomètre par résonance magnétique pour la représentation en temps réel d'un instrument médical sur une trajectoire (101), dans lequel le tomodensitomètre (1) par résonance magnétique a une commande (23), dans lequel la commande (23) est conçue pour détecter une première tranche (71) au moyen d'un codage en 3D, dans lequel la commande choisit, lors d'une représentation, la position de la première tranche (71) en fonction de la trajectoire et l'épaisseur de la tranche en fonction d'une distorsion déterminée à l'avance du tomodensitomètre (1) par résonance magnétique, de manière à ce que la trajectoire (101) soit détectée complètement par la première tranche (71) ; la commande (23) est conçue pour détecter une deuxième tranche (72) au moyen d'un codage en 3D, dans lequel la commande (23) choisit, lors d'une représentation, la position de la deuxième tranche (23) en fonction de la trajectoire (101) et l'épaisseur de la tranche en fonction d'une distorsion déterminée à l'avance du tomodensitomètre (1) par résonance magnétique, de manière à ce que la trajectoire (101) soit détectée complètement par la deuxième tranche (72) ;
dans lequel la commande (23) est conçue pour choisir la position de la première tranche (71) et de la deuxième tranche (72), de manière à ce que la première tranche (71) et la deuxième tranche (72) fassent un angle plus grand que 20 degrés, et la trajectoire (101) de l'instrument (60) médical et/ou l'instrument (60) médical se trouve dans un volume (78) de coupe de la première tranche (71) et de la deuxième tranche (72) ; la commande (23) est conçue pour produire une séquence de représentations en deux dimensions de l'instrument (60) médical dans un plan déterminé à l'avance, dans lequel la séquence comporte des représentations, qui ont été produites à partir de la première tranche (71), et des représentations, qui ont été produites à partir de la deuxième tranche (72) ; et la commande (23) est conçue pour donner la séquence de représentation en deux dimensions à un utilisateur.

2. Tomodensitomètre par résonance magnétique suivant la revendication 1, dans lequel la commande (23) du tomodensitomètre (1) par résonance magnétique est conçue pour exécuter une correction de la distorsion déterminée à l'avance de la première tranche (71) et/ou de la deuxième tranche (72).

3. Tomodensitomètre par résonance magnétique suivant la revendication 2, dans lequel la commande (23) est conçue, en outre, pour effectuer une moyenne de la première tranche (71) et/ou de la deuxième tranche (72) après la correction.

4. Tomodensitomètre par résonance magnétique suivant la revendication 3, dans lequel la commande (23) est conçue, en outre, pour effectuer la correction avec une résolution plus grande qu'une résolution de la représentation en deux dimensions.

5. Tomodensitomètre par résonance magnétique suivant l'une des revendications précédentes, dans lequel la commande (23) est conçue, en outre, pour détecter une troisième tranche contenant l'instrument médical avec un codage en 3D, dans lequel la commande (23) est conçue pour produire, lors de la production de la séquence de représentations en deux dimensions, les représentations en fonction de la troisième tranche.

6. Tomodensitomètre par résonance magnétique suivant l'une des revendications précédentes, dans lequel la trajectoire (101) est une trajectoire (101) incurvée.

7. Tomodensitomètre par résonance magnétique suivant la revendication 6, dans lequel la commande (23) est conçue pour effectuer, lors de la production de la représentation en deux dimensions, une linéarisation de la trajectoire (101) incurvée.

8. Procédé de représentations en temps réel d'un instrument (60) médical au moyen d'un tomodensitomètre (1) par résonance magnétique sur une trajectoire (101), dans lequel le tomodensitomètre (1) par résonance magnétique a une commande (23) et le procédé comporte les stades :
(S20) détection d'une première tranche (71) par le tomodensitomètre (1) par résonance magnétique au moyen d'un codage en 3D, dans lequel une position de la première tranche (71) est choisie par la commande (23) lors d'une représentation en fonction de la trajectoire (101) et l'épaisseur de tranche en fonction d'une distorsion déterminée à l'avance du tomodensitomètre (1) par résonance magnétique, de manière à ce que la trajectoire (101) soit détectée complètement par la première tranche (71),
(S30) détection d'une deuxième tranche (72) par le tomodensitomètre (1) par résonance magnétique au moyen d'un codage en 3D, dans lequel une position de la deuxième tranche (72) est choisie par la commande (23) lors d'une représentation en fonction de la trajectoire (101) et l'épaisseur de tranche en fonction d'une distorsion déterminée à l'avance du tomodensitomètre (1) par résonance magnétique, de manière à ce que la trajectoire (101) soit détectée complètement par la deuxième tranche (72) ;
dans lequel on choisit la position de la première tranche (71) et de la deuxième tranche (72), de manière à ce que la première tranche (71) et la deuxième tranche (72) fassent un angle plus grand que 20 degrés, et la trajectoire (101) de l'instrument (60) médical et/ou l'instrument (60) médical se trouve dans un volume de coupe de la première tranche (71) et de la deuxième tranche (72) ;
(S70) production par la commande (23) d'une séquence de représentations en deux dimensions de l'instrument (60) médical dans un plan déterminé à l'avance, dans lequel la séquence a des représentations, qui ont été produites à partir de la première tranche (71), et des représentations, qui ont été produites à partir de la deuxième tranche (72) ;
(S80) envoi de la séquence de représentations en deux dimensions à un utilisateur.

9. Procédé suivant la revendication 8, dans lequel le procédé a en outre le stade (S40) d'effectuer par la commande (23) une correction de la distorsion déterminée à l'avance de la première tranche (71) et/ou de la deuxième tranche (72).

10. Procédé suivant la revendication 9, dans lequel le procédé a en outre le stade (S50) d'effectuer par la commande (23), après la correction, une moyenne de la première tranche (71) et/ou de la deuxième tranche (72).

11. Procédé suivant la revendication 10, dans lequel le stade (S40) de la correction s'effectue avec une résolution plus grande qu'une résolution de la représentation en deux dimensions.

12. Procédé suivant l'une des revendications 8 à 11, dans lequel le procédé a, en outre, le stade (S60) de détection par le tomodensitomètre (1) par résonance magnétique par un codage en 3D d'une troisième tranche contenant l'instrument médical, dans lequel dans le stade de la production d'une séquence de représentations en deux dimensions, on produit les représentations en fonction de la troisième tranche.

13. Procédé suivant l'une des revendications 8 à 12, dans lequel la trajectoire (101) est une trajectoire (101) incurvée.

14. Procédé suivant la revendication 13, dans lequel, dans le stade de la production de la représentation en deux dimensions, il s'effectue une linéarisation de la trajectoire (101) incurvée.

15. Produit de programme d'ordinateur, comprenant des instructions, qui, lors de l'exécution par un tomodensitomètre (1) par résonance magnétique suivant l'une des revendications 1 à 7, font que le système (1) de tomodensitométrie par résonance magnétique exécute un procédé suivant l'une des revendications 8 à 14.
